(19) Europäisches Patentamt

European Patent Office

Office européen des brevets

(11) EP 0 546 676 B1

(12) EUROPEAN PATENT SPECIFICATION

(45) Date of publication and mention
of the grant of the patent:
**22.12.2004 Bulletin 2004/52**

(21) Application number: **92309930.3**

(22) Date of filing: **29.10.1992**

(51) Int Cl.[7]: **A61K 31/60**, A61K 31/42,
A61K 31/415, A61K 31/405,
A61K 31/19
// (A61K31/60, 31:135),
(A61K31/42, 31:135),
(A61K31/415, 31:135),
(A61K31/405, 31:135),
(A61K31/19, 31:135,
A61P29:00)

(54) **Composition comprising a tramadol material and a non-steroidal anti-inflammatory drug**

Zusammensetzung die Tramadol und ein nichtsteroides entzündungshemmenden Mittel enthält

Composition contenant du tramadol et un agent anti-inflammatoire non-stéroide

(84) Designated Contracting States:
**AT BE CH DE DK ES FR GB GR IE IT LI LU NL PT
SE**

(30) Priority: **30.10.1991 US 785137**

(43) Date of publication of application:
**16.06.1993 Bulletin 1993/24**

(73) Proprietor: **MCNEILAB, INC.
Spring House Pennsylvania 19477-0776 (US)**

(72) Inventor: **Raffa, Robert B.
Norristown, PA 19401 (US)**

(74) Representative: **Mercer, Christopher Paul et al
Carpmaels & Ransford
43, Bloomsbury Square
London WC1A 2RA (GB)**

(56) References cited:
• **BIOLOGICAL ABSTRACTS vol. 91, no. 11 , 1
June 1991, Philadelphia, PA, US; abstract no.
122708, MOROZ B. T. ET AL. 'Use of tramadol
hydrochloride in therapeutic operative dentistry
clinical investigation' page 919 ;**

Note: Within nine months from the publication of the mention of the grant of the European patent, any person may give notice to the European Patent Office of opposition to the European patent granted. Notice of opposition shall be filed in a written reasoned statement. It shall not be deemed to have been filed until the opposition fee has been paid. (Art. 99(1) European Patent Convention).

**Description**

United States Patent No. 3,652,589 discloses a class of analgesic cycloalkanol-substituted phenol esters having a basic amine group in the cycloalkyl ring. The compound (1R, 2R or 1S, 2S)-2-[(dimethylamino)methyl]-1-(3-methoxyphenyl)cyclohexanol, commonly known as tramadol, is specifically disclosed therein. A series of articles pertaining to the pharmacology, toxicology and clinical studies of tramadol are found in Arzneim. Forsch. (Drug Res.), 28(I), 114(1978). Driessen et al., Arch. Pharmacol., 341, R104 (1990) disclose that tramadol produces its analgesic effect through a mechanism that is neither fully opioid-like nor non-opioid-like. The Abstracts of the VIth World Congress on Pain, April 1-6 (1990), disclose that tramadol hydrochloride is an orally active pure agonist opioid analgesic. However, clinical experience indicates that tramadol lacks many of the typical side effects of opioid agonists, e.g., respiratory depression (W. Vogel et al., Arzneim. Forsch. (Drug Res.), 28(I), 183 (1978)), constipation (I. Arend et al., Arzneim. Forsch. (Drug Res.), 28(I), 199 (1978)), tolerance (L. Flohe et, al., Arzneim. Forsch. (Drug Res.), 28(I), 213 (1978)), and abuse liability (T. Yanagita, Arzneim. Forsch. (Drug Res.), 28(I), 158 (1978)). When given at a dose of 50 mg by rapid i.v. injection, tramadol may produce certain side effects unique to tramadol including hot flushes and sweating. Despite these side effects, tramadol's combination of non-opioid and opioid activity makes tramadol a very unique drug. Tramadol is currently being marketed by Grunenthal GMBH as an analgesic.

Opioids have for many years been used as analgesics to treat severe pain. They, however, produce undesirable side effects and as a result cannot always be given repeatedly or at high doses. The side effect problems are well documented in the literature. See, for example, J. Jaffe in "Goodman and Gilman's, The Pharmacological Basis of Therapeutics", 8th edition; Gilman et al.; Pergamon Press, New York, 1990; Chapter 22; pages 522-573 wherein it is disclosed that morphine and its congeners, e.g., codeine, hydrocodone and oxycodone, are opioid agonist analgesics that exhibit side effects such as respiratory depression, constipation, tolerance and abuse liability.

As alternatives to using opioids, non-opioids such as acetaminophen, aspirin and ibuprofen are used as analgesics. Ibuprofen. like aspirin, is not subject to the tolerance, addiction and toxicity of the opioid analgesics. However, ibuprofen, aspirin and other nonsteroidal antiinflammatory drugs (commonly referred to as NSAIDs) are only useful in relieving pain of moderate intensity, whereas the opioid analgesics are useful in relieving more intense pain; See Woodbury, D. and Fingl, E. in "The Pharmacological Basis of Therapeutics", 5th Ed.; Goodman, L. and Gilman, A., Chapter 15, (1975).

To reduce the side effect problems of opioids, opioids have been combined with other drugs including non-opioid analgesic agents, which lower the amount of opioid needed to produce an equivalent degree of analgesia. It has been claimed that some of these combination products also have the advantage of producing a synergistic analgesic effect. For example, A. Takemori, Annals New York Acad. Sci., 281, 262 (1976) discloses that compositions including combinations of opioid analgesics with drugs other than analgesics exhibit a variety of effects, i.e., subadditive (inhibitory), additive or superadditive. R. Taber et al., J. Pharm. Expt. Thera., 169(1), 29 (1969) disclose that the combination of morphine and methadone, another opioid analgesic, exhibits an additive effect. United States Patent No. 4,571,400 discloses that the combination of dihydrocodeine, an opioid analgesic, and ibuprofen, a non-opioid analgesic, provides superadditive effects when the components are within certain ratios. See also U.S. Patent Nos. 4,587,252 and 4,569,937 which disclose other ibuprofen opioid combinations. A. Pircio et al., Arch. Int. Pharmacodyn., 235, 116 (1978) report superadditive analgesia with a 1:125 mixture of butorphanol, another opioid analgesic, and acetaminophen, a non-opioid analgesic, whereas a 1:10 mixture did not show any statistically significant superadditive analgesia.

Combinations of non-opioid analgesics have also been prepared to avoid the side effects associated with opioids, and the combinations are noted to have the benefit of requiring less of each ingredient and producing superadditive effects. G. Stacher et al., Int. J. Clin. Pharmacol. Biopharmacy, 17, 250 (1979) report that the combination of non-opioid analgesics, i.e., tolmetin (another NSAID) and acetaminophen, allows for a marked reduction in the amount of tolmetin required to produce analgesia. In addition, United States Patent No. 4,260,629 discloses that an orally administered composition of acetaminophen and zomepirac, a non-opioid analgesic, in a particular weight ratio range produces a superadditive relief of pain in mammals. Furthermore, United States Patent No. 4,132,788 discloses that 5-aroyl-1-(lower)alkylpyrrole-2-acetic acid derivatives, non-opioid analgesics, when combined with acetaminophen or aspirin exhibit superadditive antiarthritic activity. However, there have been warnings against the daily consumption of non-opioid analgesic in large amounts or over long periods (see, D Woodbury and E. Fingl at page 349). In addition, ibuprofen, aspirin and some other NSAIDs may cause gastrointestinal side effects, especially if used repeatedly. See, for example, M.J.S. Langman, Am. J. Med. 84 (Suppl. 2A): 15-19, 1988; P.A. Insel in "The Pharmacological Basis of Therapeutics" 8th Ed.; Gilman, A.G. et al., Chapter 26, pp. 664-668, 1990.

B.T. Moroz et al. in Biological Abstracts vol. 91, No. 122708 (1991) and Curr. Ther. Res. vol 49(3), pages 371-375 (1991) describe the use of tramadol hydrochloride for the treatment of dental pain. Analgesia was found to be most effective when a single dose of tramadol hydrochloride not exceeding 2.5mg/kg was combined with a single oral dose of 400mg of the nonsteroidal anti-inflammatory drug ibuprofen.

The prior art, however, does not disclose that tramadol, an "atypical" opioid analgesic, can or should be combined with another analgesic to lessen the side effects of each or to yield a composition comprising a tramadol material and another analgesic that exhibits superadditive analgesia.

It has now been found that a tramadol material which includes various forms of tramadol as defined hereinafter can be combined with nonsteroidal antiinflammatory drugs (hereinafter NSAIDs) to produce analgesia. The combination employs lesser amount of both the tramadol material and the NSAID than would be necessary to produce the same amount of analgesia if either was used alone. By using lesser amounts of both drugs the side effects associated with each are reduced in number and degree. Surprisingly, the compositions comprising the tramadol material and one or more NSAIDs have been found to exhibit synergistic analgesic effects when combined in certain ratios. The present invention provides a pharmaceutical composition comprising a tramadol material and diclofenac. The compositions according to this invention may also be useful in treating tussive conditions.

In the accompanying drawings:-

The present invention is generally directed to compositions comprising a tramadol material and diclofenac. The tramadol material is any one of (1R, 2R or 1S, 2S)-2-[(dimethylamino)methyl]-1-(3-methoxyphenyl)-cyclohexanol (tramadol), its N-oxide derivative ("tramadol N-oxide"), and its O-desmethyl derivative ("O-desmethyl tramadol") or mixtures thereof. It also includes the individual stereoisomers, mixtures of stereoisomers, including the racemates, pharmaceutically acceptable salts of the amines, such as the hydrochloride salt, solvates and polymorphs of the tramadol material. Tramadol is commercially available from Grunenthal or may be made by the process described in United States Patent No. 3,652,589, which is herein incorporated by reference.

Tramadol N-oxide is prepared by treating tramadol as a free base with an oxidizing agent, _e.g._, hydrogen peroxide (30%), in an organic solvent, _e.g._, methanol or isopropanol, with, but preferably without heating. See, "Reagents For Organic Synthesis", 1, 471, Fieser & Fieser eds., Wiley N.Y; (1987), B. Kelentey _et al._, _Arzneim. Forsch._, 7, 594 (1957). With heating, the reaction takes about 1 hour, whereas without heating the reaction takes about 3 days. Following the oxidation, the mixture is treated with an agent, _e.g._ PtO$_2$ or preferably Pt/C, for about a day, to destroy the excess hydrogen peroxide. The mixture is filtered, followed by the evaporation of the filtrate and then the residue is recrystallized from an organic solvent mixture, _e.g._, methylene chloride/ethyl acetate.

O-desmethyl tramadol is prepared by treating tramadol as a free base under O-demethylating reaction conditions, _e.g._, reacting it with a strong base such as NaH or KH, thiophenol and diethylene glycol (DEG) with heating to reflux. See, Wildes _et al._, _J. Org. Chem._, 36, 721 (1971). The reaction takes about an hour, followed by cooling and then quenching in water of the reaction mixture. The quenched mixture is acidified, extracted with an organic solvent such as ethyl ether, basified and then extracted with a halogenated organic solvent such as methylene chloride. The extract is then dried and the solvent evaporated to yield the O-desmethyl product, which may then be short-path distilled, converted to its corresponding salt, _e.g._, treated with an acidified (HCl/ethanol) solution, and recrystallized from an organic solvent mixture, _e.g._, ethanol/ethyl ether.

NSAIDs are non-opioid analgesics characterized in that they are nonsteroidal drugs which act as antiinflammatory, analgesic and anti-pyretic agents. This class of drugs is well known in the art. See for example, Goodman, L. and Gilman, A., _supra_, in Chapter 26 (1990). These drugs share certain therapeutic actions and side effects. Within this broad class of drugs are salicylates, such as aspirin; pyrazolone derivatives such as phenylbutazone, onyphenbutazone, antipyrine, aminopyrine, dipyrone and apazone; indomethacin; sulindac; fenamates such as mefenamic, meclofenamic, flufenamic, tolfenamic and etofenamice acids; aryl acetic acid and propionic acid compounds such as alphamethyl-4-(2-thienylcarbonyl) benzene acetic acid (generic name suprofen); 4,5-diphenyl-2-oxazole propionic acid (generic name oxprozin); rac-6-chloro-alphamethyl-carbazole-2-acetic acid (generic name carprofen); 2-(3-phenyloxyphenyl)-propionic acid, particularly the calcium salt dihydrate thereof (these compounds being referred to generically as fenoprofen and fenoprofen calcium); 2-(6-methoxy-2-naphthyl) propionic acid (generic name naproxen; the generic name of the sodium salt is naproxen sodium); 4-(1,3-dihydro-1-oxo-2H-isoindol-2-yl)-α-methylbenzene acetic acid (generic name indoprofen); 2-(3-benzoylphenyl)propionic acid (generic name ketoprofen); and 2-(2-fluoro-4-biphenylyl) propionic acid (generic name flurbiprofen) and 1-5-(4-methylbenzoyl)-1H-pyrrole-2-acetic acid (generic name tolmetin). Also included within NSAIDs are compounds within the class including sodium 5-(4-chlorobenzoyl)-1,4-dimethyl-1H-pyrrole-2-acetate dihydrate (generically referred to as zomepirac sodium); 4-hydroxy-2-methyl-N-(2-pyridyl-2H-1,2-benzothiazine-3-carboxamide-1,1-dioxide (generic name piroxicam); 2',4'-difluoro-4-hydroxy-3-biphenylcarboxylic acid (generic name diflunisal) or 1-isopropyl-7-methyl-4-phenyl-2(1H)-quinozolinone (generic name proquazone), phenylacetic acid derivatives such as diclofenac; etodolac and nabumetone.

The NSAID which is used in the present invention is diclofenac.

The NSAID and the tramadol material are generally present in a weight ratio of tramadol material to NSAID from 1:1 to 1:200. Certain ratios result in a composition which exhibits synergistic analgesic effects. For example, in a composition comprising a tramadol material and an NSAID, the ratio of the tramadol material: NSAID is preferably from 1:1 to 1:200; and, more preferably, from 1:2 to 1:200. The most preferred ratios are from 1:2 to 1:20. Compositions of a tramadol material and NSAID within these weight ratios have been shown to exhibit synergistic analgesic effects.

Pharmaceutical compositions comprising the tramadol material and the NSAID as the active ingredients in an intimate admixture with a pharmaceutical carrier can be prepared according to conventional pharmaceutical compounding techniques. The carrier may take a wide variety of forms depending on the form of preparation desired for administration, e.g., intravenous, oral or parenteral. The composition may also be administered by means of an aerosol. In preparing the compositions in an oral dosage form, any of the usual pharmaceutical media may be employed. For example, in the case of oral liquid preparations (such as suspensions, elixirs and solution), water, glycols, oils, alcohols, flavoring agents, preservatives, coloring agents and the like may be used. In the case of oral solid preparations (such as, for example. powders, capsules and tablets), carriers such as starches, sugars, diluents, granulating agents, lubricants, binders, disintegrating agents and the like. may be used. Because of their ease in administration, tablets and capsules represent the most advantageous oral dosage unit form, in which case solid pharmaceutical carriers are obviously employed. If desired, tablets may be sugar-coated or enteric-coated by standard techniques. For parenterals, the carrier will usually comprise sterile water, though other ingredients, for example, to aid solubility or for preservative purposes, may be included. Injectable suspensions may also be prepared, in which case appropriate liquid carriers, suspending agents and the like may be employed. The pharmaceutical compositions will generally be in the form of a dosage unit, e.g., tablet, capsule, powder, injection, teaspoonful and the like, containing from 0.1 to about 800 mg/kg, and preferably from about 0.3 to 200 mg/kg of the active ingredients. However, it will be appreciated that the precise dose of the active ingredients will vary depending upon the particular NSAID and tramadol material being used.

The following experimental reference examples are intended to be a way of illustrating but not limiting the invention.

Example 1: Preparation of the Combined Doses of Tramadol and Ibuprofen

The preparation of different ratios of a tramadol/ibuprofen combination was effected by preparing solutions having concentrations expressed in $mg_{drugs}$ per 10 mL of distilled water. For example, 40 mg of tramadol as the free base and 40 mg of ibuprofen as the free base were added to 10 mL of water with 2 drops of TWEEN 80, a pharmacological dispersant, manufactured by Fisher Scientific Company, to yield the highest concentration of the tramadol/ibuprofen 1:1 (40mg:40mg) ratio. Each concentration of the ratio was prepared separately in a similar manner and injected in a volume of 10 mL/kg per mouse. Similarly, the other ratios of tramadol/ibuprofen listed in Table 1 were prepared at the various concentrations.

Example 2: Preparation of the Combined Doses of Tramadol N-oxide and Ibuprofen

First, tramadol N-oxide was prepared as set forth hereinafter. Tramadol hydrochloride (0.5 mol) was converted to its free base in basified water (pH >9) and then extracted with ether. The ether was evaporated to yield the crystalline hydrate of tramadol. The solid was then heated with steam under a high vacuum to remove as much water as possible to yield 131.5 g of material. The material was dissolved in methanol (500 mL) and 65 g of 30% $H_2O_2$ was added. The solution was stirred for 3 hours and then an additional 65 g of the 30% $H_2O_2$ was added. The reaction was stirred for 2.5 days at room temperature. Approximately 10 mg of $PtO_2$ on carbon (use of Pt/C is suggested for its ease of removal) was added to the reaction mixture. and very gentle foaming took place. An additional 10 mg of $PtO_2$ was added and the reaction mixture was stirred overnight and then filtered thru a filter aid. The filtrate was concentrated under vacuum while being heated to a temperature of <40°C. The residue was taken up in methylene chloride. Since the methylene chloride solution contained some colloidial platinum, the solution was diluted with ethyl acetate to 1 L and filtered through a nylon filter membrane (0.45 μ pore size) to yield a clear colorless filtrate. The filtrate was concentrated to 600 mL, and then ethyl acetate was added continuously to maintain a volume of 800 mL while the solution was heated until a vapor temperature of 74°C was reached. The solution was then cooled to room temperature. The solid was collected by filtration, washed with ethyl acetate and dried in vacuo to yield 126.6 g of the tramadol N-oxide (mp. 159.5-160°C).

| C16H25NO3 Theor. | C, 68.78; | H, 9.27; | N, 5.01 |
|---|---|---|---|
| Found | C, 68.65; | H, 9.22; | N, 4.99 |

The preparation of different ratios of a tramadol N-oxide/ibuprofen combination is effected by preparing solutions having concentrations expressed in $mg_{drugs}$ per 10 mL of distilled water. For example, 40 mg of tramadol as the free base and 40 mg of ibuprofen as the free base is added to 10 mL of water with 2 drops of TWEEN 80, a pharmacological dispersant, manufactured by Fisher Scientific Company, to yield the highest concentration of the tramadol N-oxide/ibuprofen 1:1 (40mg:40mg) ratio. Each concentration of the ratio is prepared separately in a similar manner.

Example 3: (-) and (+) Enantiomers of O-Desmethyl Tramadol: Their Syntheses and the Preparation of Doses of O-Desmethyl Tramadol- with Ibuprofen

First, O-desmethyl tramadol was prepared as set forth hereinafter. Diethylene glycol (125 mL) was added with cooling to potassium hydride (9.5 g) with the temperature being maintained at <50°C. To the solution was added thiophenol (10 mL) dissolved in diethylene glycol (25 mL), and then (-)-tramadol as the free base (9.3 g) in diethylene glycol (50 mL) was added. The final reaction mixture was heated slowly to reflux for 45 minutes. The mixture was cooled and then quenched into water. The pH was adjusted to about 3, and the mixture was extracted with ethyl ether. The pH was readjusted to about 8 and the resulting mixture was extracted 5 more times with methylene chloride. The extract was dried and the methylene chloride was evaporated to yield 4.6 g of the title compound as an oil. The oil was distilled (Kugelrohr), dissolved in tetrahydrofuran and treated with an ethanol/HCl solution to give 2.3 g of the salt . The salt was recrystallized from ethanol/ethyl ether and dried to yield 1.80 g of the salt of the (-) enantiomer of O-desmethyl tramadol (mp. 242-3°C), $[\alpha]_D^{25}$ = -32.9(C=1, EtOH).

| $C_{15}H_{23}NO_2 \cdot HCl$ Theor.: | C, 63.04; | H, 8.46; | N, 4.90 |
|---|---|---|---|
| Found: | C, 63.00; | H, 8.51; | N, 4.94 |

To prepare the (+) enantiomer of the title compound, the reaction was run under the same conditions except that (+)-tramadol as the free base was used instead of the (-)-tramadol to yield 2.8 g of the (+) enantiomer of O-desmethyl tramadol (mp. 242-3°C) $[\alpha]_D^{25}$ =+32.2 (C=1, EtOH).

| $C_{15}H_{23}NO_2 \cdot HCl$ Theor.: | C, 63.04; | H, 8.46; | N, 4.90 |
|---|---|---|---|
| Found: | C, 63.14; | H, 8.49; | N, 4.86 |

The preparation of different ratios of a O-desmethyl/ibuprofen combination is effected by preparing solutions having concentrations expressed in $mg_{drugs}$ per 10 mL of distilled water. For example, 40 mg of O-desmethyl as the free base and 40 mg of ibuprofen as the free base were added to 10 mL of water with 2 drops of TWEEN 80, a pharmacological dispersant, manufactured by Fisher Scientific Company, to yield the highest concentration of the O-desmethyl tramadol/ibuprofen 1:1 (40mg:40mg) ratio. Each concentration of the ratio is prepared separately in a similar manner and injected in a volume of 10 mL/kg per mouse.

Example 4: Analgesic Activity

Male CD1 mice (weighing from 18-24 g) were utilized in determining the analgesic effects associated with the compositions of the invention. The mice were all dosed orally with tramadol hydrochloride (calculated as the base), which was completely dissolved in distilled water, and ibuprofen (calculated as the base), which was completely dissolved in distilled water or in distilled water containing 2% by volume of Tween 80 containing 100% polysorbate 80. The dosing volume was 10 mL/kg.

The procedure used in detecting and comparing the analgesic activity of different classes of analgesic drugs for which there is a good correlation with human efficacy is the prevention of acetylcholine-induced abdominal constriction in mice (H. Collier et al., Br. J. Pharmacol., 32, 295 (1968)).

Mice, intubated with various doses of tramadol hydrochloride alone, ibuprofen alone, combined doses of tramadol hydrochloride and ibuprofen, or vehicle such as distilled water, or distilled water containing 2% by volume of Tween 80, were injected intraperitoneally with a challenge dose of acetylcholine bromide. The acetylcholine was completely dissolved in distilled water at a concentration of 5.5 mg/kg and injected at the rate of 0.20 mL/20 g. For scoring purposes an "abdominal constriction" was defined as a contraction of the abdominal musculature accompanied by arching of the back and extension of the limbs. The mice were observed 10 minutes for the presence or absence of the abdominal constriction response beginning immediately after receiving the acetylcholine dose, which was 30 minutes after receiving the oral administration of tramadol hydrochloride, ibuprofen, combined doses of tramadol hydrochloride and ibuprofen, or vehicle. Each mouse was used only once.

The analysis of possible superadditivity for the compositions at each fixed ratio was determined as disclosed by R. J. Tallarida et al., Life Sci., 45, 947 (1989). This procedure involved the determination of the total amount in the mixture that is required to produce a specified level of effect, such as 50% ($ED50_{mix}$), and the corresponding total amount that would be expected under simple additivity ($ED50_{add}$). Where it was established that $ED50_{mix} < ED50_{add}$ for a specific fixed-ratio, then that composition ratio was superadditive. Both the quantities $ED50_{mix}$ and $ED50_{add}$ were random variables; $ED50_{mix}$ was estimated from the dose-response curve for a specific fixed-ratio; $ED50_{add}$ was obtained by combining the ED50 estimates for the two drugs under additivity. $ED50_{mix}$ was then compared to $ED50_{add}$

via a Student's t-test. The ED50 value for tramadol hydrochloride alone was 5.5(4.8-6.4) mg/kg. The ED50 value for ibuprofen alone was 33.5 (24.1-46.5) mg/kg.

The interaction between tramadol and ibuprofen was determined at precise dosage ratios of tramadol hydrochloride and ibuprofen. Multiple (typically 4-6) coded doses of each selected combination were studied for analgesic effectiveness after 30 minutes using an experimental design which permitted the complete randomization of the separate dosage forms tested.

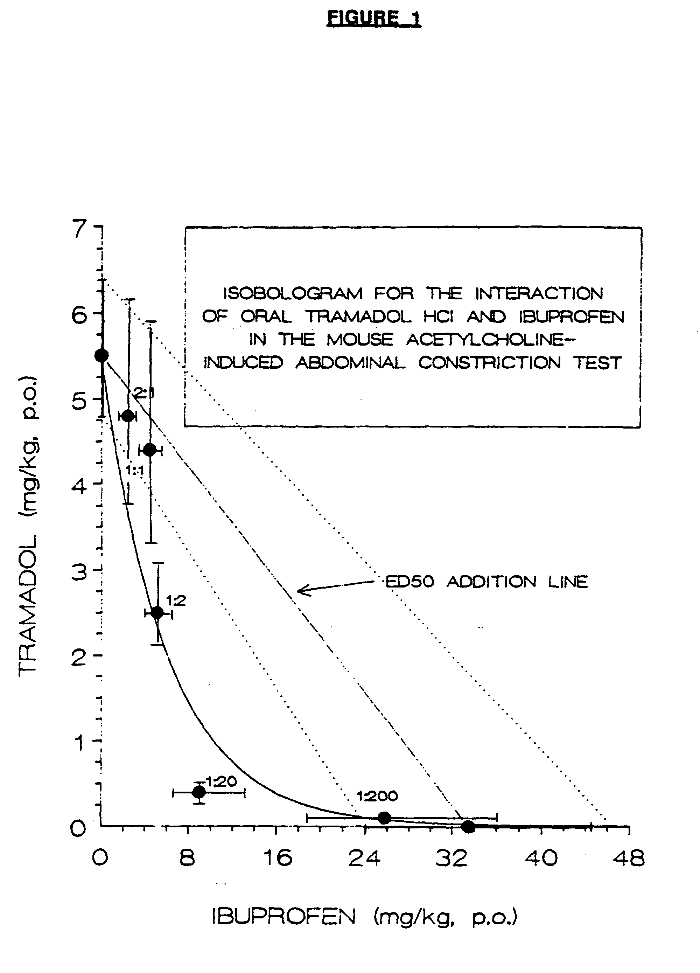

The interaction of tramadol hydrochloride and ibuprofen on the acetylcholine-induced abdominal constriction in mice was demonstrated by the data in Table I and is shown in the Loewe isobologram, Figure I, (see, S. Loewe, Pharm. Rev., 9; 237 (1957) regarding the preparation and basis of an isobologram). In Figure 1, the diagonal line joining the ED50 values of the two drugs given separately represents the simple additivity of effects at different component ratios. The dotted lines adjacent to the diagonal line define the 95% confidence interval. ED50 values falling under the curve (between the line and the origin) indicate superadditivity, i.e., unexpected enhancement of effects. The diagonal dashed lines radiating from the origin represent the dose ratios of ibuprofen to tramadol hydrochloride used in mice receiving the combined drug dosages. The bars through the ED50 points for the tramadol and ibuprofen composition represent the 95% confidence intervals of the ED50 value. The experimental data as represented in Figure I establish that compositions having a ratio of tramadol to ibuprofen from about 1:1 to 1:200 (represented by the curved line) give unexpectedly enhanced activity since $ED50_{mix}$ is less than $ED50_{add}$.

It is expected that based on these results, other NSAIDs, and particularly the propionic acid derivatives, when combined with a tramadol material, will produce similar synergistic results.

TABLE I

| TRAMADOL:IBUPROFEN | | | | | |
|---|---|---|---|---|---|
| DRUG COMBINATIONS | DOSE (mg/kg, p.o.) | | | $ED_{50}$ at 30 min (95% CI's) | |
| Tramadol:Ibuprofen) | Tramadol | Ibuprofen | analgesia | Tramadol | Ibuprofen |
| tramadol only | 2 | 0 | 3/15 | | |
| | 3 | 0 | 4/15 | | |
| | 4 | 0 | 14/45 | | |
| | 6 | 0 | 20/45 | 5.5 | |
| | 8 | 0 | 40/60 | (4.8-6.4) | |
| | 10 | 0 | 15/15 | | |
| | 16 | 0 | 14/15 | | |
| 2:1 | 2.5 | 1.25 | 2/15 | | |
| | 5 | 2.5 | 7/15 | 4.8 | 2.4 |
| | 10 | 5 | 14/15 | (3.8-6.2) | (1.9-3.1) |
| 1:1 | 1.25 | 1.25 | 2/15 | | |
| | 2.5 | 2.5 | 3/15 | 4.4 | 4.4 |
| | 5 | 5 | 6/15 | (3.3-5.9) | (3.3-5.9) |
| | 10 | 10 | 15/15 | | |
| 1:2 | 1.25 | 2.5 | 1/15 | | |
| | 2.5 | 5 | 7/15 | 2.5 | 5.1 |
| | 5 | 10 | 14/15 | (2.1-3.1) | (4.1-6.3) |
| | 10 | 20 | 15/15 | | |
| 1:20 | 0.15625 | 3.125 | 2/15 | | |
| | 0.3125 | 6.25 | 7/15 | | |
| | 0.625 | 12.5 | 8/15 | 0.4 | 9.0 |
| | 1.25 | 25 | 26/30 | (0.3-0.6) | (6.3-12.8) |
| | 2.5 | 50 | 13/15 | | |
| | 5 | 100 | 15/15 | | |

TABLE I   (continued)

| TRAMADOL:IBUPROFEN | | | | | |
|---|---|---|---|---|---|
| DRUG COMBINATIONS | DOSE (mg/kg, p.o.) | | | $ED_{50}$ at 30 min (95% Cl's) | |
| Tramadol:Ibuprofen) | Tramadol | Ibuprofen | analgesia | Tramadol | Ibuprofen |
| 1:200 | 0.05 | 10 | 3/15 | | |
| | 0.1 | 20 | 6/15 | | |
| | 0.2 | 40 | 9/15 | 0.1 | 25.8 |
| | 0.4 | 80 | 13/15 | (0.1-0.2) | (18.4-36.2) |
| | 0.8 | 160 | 15/15 | | |
| Ibuproten only | 0 | 10 | 2/15 | | |
| | 0 | 20 | 4/15 | | |
| | 0 | 40 | 8/15 | | 33.5 |
| | 0 | 80 | 13/15 | | (24.1-46.5) |
| | 0 | 160 | 14/15 | | |

## Claims

1.  A pharmaceutical composition comprising a tramadol material and diclofenac.

2.  The pharmaceutical composition of claim 1 wherein the tramadol material is tramadol hydrochloride.

3.  The pharmaceutical composition of claim 2 wherein the tramadol hydrochloride is racemic.

4.  The pharmaceutical composition of any preceding claim further comprising a pharmaceutically acceptable carrier.

5.  A product comprising a tramadol material and diclofenac, as a combined preparation for simultaneous, separate or sequential use in the treatment of a pharmacological condition.

## Patentansprüche

1.  Pharmazeutische Zusammensetzung, die ein Tramadol-Material und Diclofenac umfaßt.

2.  Pharmazeutische Zusammensetzung nach Anspruch 1, wobei das Tramadol-Material ein Tramadolhydrochlorid ist.

3.  Pharmazeutische Zusammensetzung nach Anspruch 2, wobei das Tramadolhydrochlorid razemisch vorliegt.

4.  Pharmazeutische Zusammensetzung nach einem der vorangehenden Ansprüche, die weiterhin einen pharmazeutisch zulässigen Träger umfaßt.

5.  Produkt, das ein Tramadol-Material und Diclofenac als ein Kombinationspräparat zur zeitgleichen, getrennten oder sequentiellen Verwendung bei der Behandlung eines pharmakologischen Zustands umfaßt.

## Revendications

1.  Composition pharmaceutique comprenant du tramadol et du déclofenac.

2.  Composition pharmaceutique de la revendication où le tramadol est du chlorhydrate de tramadol.

3.  Composition pharmaceutique de la revendication 2 où le chlorhydrate de tramadol est racémique.

**4.** Composition pharmaceutique de toute revendication précédente comprenant de plus un support pharmaceutiquement acceptable.

**5.** Produit comprenant du tramadol et du déclofenac, en tant que préparation combinée pour usage simultané, séparé ou séquentiel dans le traitement d'une condition pharmacologique.

## FIGURE 1

ISOBOLOGRAM FOR THE INTERACTION
OF ORAL TRAMADOL HCl AND IBUPROFEN
IN THE MOUSE ACETYLCHOLINE-
INDUCED ABDOMINAL CONSTRICTION TEST

ED50 ADDITION LINE

TRAMADOL (mg/kg, p.o.)

IBUPROFEN (mg/kg, p.o.)